# EUROPÄISCHE PATENTSCHRIFT

(11) **EP 0 843 536 B1**
(45) Veröffentlichungstag und Bekanntmachung des Hinweises auf die Patenterteilung: **28.06.2000**
(21) Anmeldenummer: 95932009.4
(22) Anmeldetag: 08.09.1995
(51) Int. Cl.: A61B 17/34

(54) **OPERATIONSINSTRUMENT**
SURGICAL INSTRUMENT
INSTRUMENT CHIRURGICAL

(30) Priorität: 14.09.1994 DE 4432673
(43) Veröffentlichungstag der Anmeldung: 27.05.1998
(73) Patentinhaber: Karl Storz GmbH & Co. KG, 78532 Tuttlingen (DE)
(72) Erfinder: KLEMM. Bernd, 79224 Umkirch (DE)
(74) Vertreter: Schmitt, Hans, Dipl.-Ing.
(86) Internationale Anmeldenummer: EP9503525
(87) Internationale Veröffentlichungsnummer: WO9608204

(56) Entgegenhaltungen:
- EP-A- 0 604 197
- WO-A-93/10837
- WO-A-93/17626
- US-A- 5 224 951
- US-A- 5 256 147

## Beschreibung

Die Erfindung betrifft ein Operationsinstrument, das mit seinem distalen Endbereich in das Körperinnere eines Patienten einzuführen oder einzustechen ist und eine äußere Trokarhülse sowie einen darin einführbaren und verschieblich geführten Trokardorn aufweist, wobei der Trokardorn in einer Schiebeendstellung mit seiner distalen Trokardornspitze über den zur Hülsen-Längsachse schrägen Endbereich der Trokarhülse übersteht.

Operationsinstrumente der eingangs erwähnten Art werden in der minimal-invasiven Chirurgie für die Diagnose und Operation am lebenden Körper eines Patienten verwendet.

So wird zur Durchführung einer intraabdominalen Operation zunächst der Bauchraum des Patienten mittels einer Insuflationsnadel durchstochen. Diese hohle Insuflationsnadel ist über eine Schlauchverbindung an eine Gasquelle angeschlossen, um den Bauchraum des Patienten mit Gas füllen und entsprechend aufweiten zu können. Nach dem Aufweiten des Bauchraumes wird das Operationsinstrument praktisch blind in die Körperhöhle eingestochen, wobei der Trokardorn in die Trokarhülse eingesetzt ist und mit seiner Trokardornspitze über das distale Hülsenende übersteht.

Nach dem Einstechen des Operationsinstrumentes kann der Trokardorn aus der Trokarhülse entfernt und gegen optische Beobachtungsinstrumente, Greifer, Fadenhalter, Faßzangen oder dergleichen Operationsinstrumente ausgetauscht werden.

Der Trokardorn ist an seiner Trokardornspitze meist kegelförmig ausgebildet, um das Durchstechen der Gewebeschichten zu erleichtern. Man hat auch schon Trokardorne mit mehrkantigen Trokardornspitzen geschaffen, die wie ein Messer wirken und das zähe Peritoneum (Bauchfell) praktisch aufschneiden, jedoch haben diese meist dreikantigen Trokardornspitzen den Nachteil, daß sie im Falle einer unbeabsichtigten Verletzung scharfe und große Wunden verursachen.

Um nach dem Durchstechen des Peritoneums mit Hilfe des Trokardorns auch die Trokarhülse besser nachschieben zu können, ist es bereits bekannt, das distale Ende der Trokarhülse schräg zu ihrer Hülsen-Längsachse anzuordnen. Der abgeschrägten Hülsenöffnung der Trokarhülse wird vom Gewebe nämlich ein geringerer Widerstand entgegengesetzt, so daß die Trokarhülse besser rutschen kann.

Beim Einstechen des Trokardorns ist das sogenannte Zeltphänomen bekannt, wonach das zähe Peritoneum auch vor dem spitzen Trokardorn nach innen zurückweicht und zeltförmig durchbiegt, bevor sich die gewünschte Stichöffnung bildet. Dabei kann die Trokardornspitze in den Bereich der Bauchorgane kommen, was die Gefahr erheblicher Verletzung für den Patienten wesentlich erhöht.

Ein zu tiefes Einstechen des in die Trokarhülse eingeschobenen Trokardorns in das Körperinnere kann schließlich zu lebensgefährlichen Verletzungen innerer Organe oder Blutgefäße durch die Trokardornspitze führen.

Aus der US-PS 5 224 951 ist bereits ein Operationsinstrument bekannt, welches eine in einen Trokar einsetzbare Durchstecheinrichtung hat. Diese Durchstecheinrichtung weist eine äußere Schneidehülse auf, in der ein Schutzstab verschieblich geführt ist. Das distale Ende der äußeren Schneidehülse ist derart schräg zur Hülsenlängsachse angeordnet, daß der distale Hülsenstirnrand zu einer in Einstechrichtung vorderen doppelschneidigen Einstechspitze ausläuft. Sobald die Durchstecheinrichtung des vorbekannten Operationsinstrumentes auf das Körpergewebe auftrifft, kann sich der Schutzstab geringfügig in das Hülseninnere bewegen, so daß die Einstechspitze freigegeben ist. Dabei weist der in der Praxis als stumpfes Plastikteil ausgestaltete Schutzstab an seinem distalen Ende zwei abgeschrägte Stirnflanken auf, die ein Ausstanzen des Gewebes durch den scharfen Hülsenrand der äußeren Schneidehülse sowie ein Eindringen ausgestanzter Gewebeteile in das Hülseninnere verhindern sollen.

Da bei dem vorbekannten Operationsinstrument gemäß der US-PS 5 224 951 die Schneidehülse an ihrem distalen Hülsenende schräg zu einer Einstechspitze zuläuft und für einen Trokar vorgesehen ist, dessen distales Ende rechtwinklig zur Trokar-Längsachse angeordnet ist, muß auch hier das distale Ende der Schneidehülse weit über das benachbarte Ende des Trokars nach außen vorgeschoben werden. Das durch die zweischneidige Einstechspitze ohnehin schon hohe Verletzungsrisiko wird daher noch zusätzlich erhöht.

In der WO-A-93/17626 ist ein Operationsinstrument beschrieben, bei dem der in einer Trokarhülse verschieblich geführte Trokardorn mit einer am proximalen Ende der Trokarhülse angeordneten Rückholeinrichtung zusammenwirkt, welche den Trokardorn während des Durchstoßens durch das Gewebe in seiner vorgeschobenen Operationsstellung hält und ihn in das Hülseninnere der Trokarhülse zurückzieht, sobald der Trokardorn entlastet wird. Die in einzelnen Ausführungsformen gemäß WO-A-93/17626 auch exzentrisch zur Trokardorn-Längsachse angeordnete Spitze des Trokardorns muß jedoch weit über das rechtwinklig zur Hülsenlängsachse angeordnete distale Stirnende der Trokarhülse in die Einstechposition vorgeschoben werden, wodurch sich auch hier ein unnötig langer Schiebeweg und ein entsprechend hohes Verletzungsrisiko beim Einstechen des Trokardorns und beim Nachschieben der Trokarhülse ergibt.

Aus der US-PS 5 256 147 kennt man bereits ein Operationsinstrument, bei welchem dem in einer Trokarhülse verschieblich geführten Trokardorn mehrere austauschbare Trokardornspitzen zugeordnet sind. Der Trokardorn des vorbekannten Operationsinstrumentes weist dazu einen Trokardornschaft auf, an dem jeweils eine Trokardornspitze lösbar befestigbar ist. Diese Trokardornspitze ist in der in die Trokarhülse eingeschobenen Gebrauchsstellung sicher am Trokardornschaft gehalten und steht bereichsweise über das rechtwinklig zur Hülsenlängsachse orientierte Hülsenende der Trokarhülse vor. Das vorbekannte Operationsinstrument gemäß der US-PS 5 256 147 weist u.a. auch Trokardornspitzen mit abgeschrägtem Spitzenenden auf, jedoch sind diese Trokardornspitzen ebenfalls weit über die stirnseitige Hülsenöffnung der Trokarhülse vorzuschieben, was für den Patienten die erwähnten Risiken mit sich bringt.

In der EP 0 604 197 A2 ist bereits ein Operationsinstrument mit einer Trokarhülse beschrieben, bei der die distale, dem Patienten zugewandte Hülsenöffnung mittels eines Schutzschildes verschlossen ist. Dieses Schutzschild wird aus mehreren, etwa kegelförmig angeordneten Schildteilen gebildet, die beim Vorschieben eines Trokardornes nach außen springen und die Hülsenöffnung zwischen sich freigeben. Auch hier muß der Trokardorn weiter über die Hülsenöffnung in seine Einstechposition vorgeschoben werden, damit seine Trokardornspitze auch über die seitlichen Schildteile des Schutzschildes vorsteht.

Aus der WO-A-93/10837 kennt man bereits ein Operationsinstrument, welches eine Kombination einer äußeren Trokarhülse mit einer darin verschieblich geführten inneren Nadel oder Kanüle darstellt. Während die Trokarhülse auch hier eine rechtwinklig zur Hülsen-Längsachse orientierte Hülsenöffnung hat, weist die Nadel oder Kanüle ein schräg zur ihrer Längsachse angeordnetes distales Stirnende auf. Um das Verletzungsrisiko zu reduzieren, das mit der über die Hülsenöffnung der Trokarhülse weit vorstehende Nadelspitze verbunden ist, ist in der WO-A-93/10837 vorgesehen, daß die Nadel oder Kanüle bei einer Druckentlastung automatisch in das Hülseninnere der Trokarhülse zurückgefahren wird. Dadurch kann jedoch nicht vermieden werden, daß die weit über die Trokarhülse vorstehende Nadelspitze solange zu unerwünschten Verletzungen führt, bis sie sich im Hülseninneren befindet.

Es besteht daher insbesondere die Aufgabe, ein Operationsinstrument der eingangs erwähnten Art zu schaffen, dessen Anwendung eine wesentlich geringere Verletzungsgefahr für den Patienten beinhaltet.

Die erfindungsgemäße Lösung dieser Aufgabe besteht bei dem Operationsinstrument der eingangs erwähnten Art insbesondere darin, daß die Trokardornspitze gegenüber der Trokardorn-Längsachse exzentrisch ausgebildet und im Bereich des distalen, dem Patienten zugewandten Endes der Trokarhülse angeordnet ist.

Die exzentrische Anordnung der Trokardornspitze erlaubt es, den Abstand zwischen dem distalen Spitzenende des Trokardorns und dem entsprechenden Hülsenende der Trokarhülse vergleichsweise kurz zu halten. Durch die exzentrische Anordnung der Trokardorn-spitze wird somit der beim Einstechen des Trokardorns und beim Nachschieben der Trokarhülse erforderliche Schiebeweg wesentlich verkürzt. Da zum Durchstechen beispielsweise der Bauchdecke des Patienten (des Peritoneums) mit Hilfe des erfindungsgemäßen Operationsinstrumentes ein geringerer Schiebeweg erforderlich ist, sinkt auch die Gefahr einer unbeabsichtigten Verletzung der inneren Organe und Blutgefäße des Patienten.

Damit der Trokardornspitze vom Gewebe beim Durchstechen ein möglichst geringer Widerstand entgegengesetzt wird, sieht eine bevorzugte Ausführungsform gemäß der Erfindung vor, daß der distale Endbereich des Trokardorns im wesentlichen als eine Verschneidung oder Verschneidungskurve zwischen einem etwa in Hülsen-Längsrichtung orientierten Zylinder und einer Kegelform ausgebildet ist. Die dem Patienten zugewandte, stirnseitige Oberfläche ist daher auch bei dem erfindungsgemäßen Trokardorn im wesentlichen an einen Oberflächenbereich einer Kegelform angepaßt.

Bei der Durchführung herkömmlicher Operationsmethoden, bei denen nach dem Aufweiten des Bauchraumes das aus Trokarhülse und Trokardorn bestehende Operationsinstrument durch die Gewebeschichten des Patienten praktisch blind durchgestochen wird, ist es zweckmäßig, wenn die Kegelspitze des an den Oberflächenbereich einer Kegelform angepaßten Trokardorns außerhalb des Trokarquerschnitts oder etwa auf dem Trokardorn-Umfang angeordnet ist.. Insbesondere wenn die Trokardornspitze etwa auf dem Trokardorn-Umfang angeordnet ist, steht diese auch hier punktförmig spitz zulaufende Trokardornspitze nur wenig über das distale Ende der Trokarhülse über.

Eine vorteilhafte Weiterbildung gemäß der Erfindung sieht vor, daß die Trokarspitze innerhalb des Trokardornquerschnitts und vorzugsweise mit Abstand von der Trokar-Längsachse einerseits und dem Trokardorn-Umfang andererseits angeordnet ist. Eine solche, sowohl gegenüber der Trokardorn-Längsachse als auch gegenüber dem Trokardorn-Umfang versetzte Trokardorn-Spitze erlaubt die Ausgestaltung einer vergleichsweise flachen SpitzenEbene, was insbesondere bei kleineren Trokaren von Vorteil sein kann.

DieTrokardornspitzedeserfindungsgemäßenOperationsinstrumentes kann punktförmig spitz zulaufen. Möglich ist aber auch, daß die Trokardornspitze schneidenförmig ausgebildet oder endseitig geringfügig abgerundet ist. Insbesondere mit einer endseitig geringfügig abgerundeten Trokardornspitze läßt sich das Verletzungsrisiko noch zusätzlich reduzieren.

Eine bevorzugte Ausführungsform gemäß der Erfindung sieht vor, daß am Trokardorn und/oder an der Trokarhülse wenigstens ein in zumindest einer Schiebeendstellung wirksames Sicherungsmittel zur Sicherung der Relativposition von Trokarhülse und Trokardorn in deren Umfangsrichtung vorgesehen ist. Bei dieser Ausführungsform wir die Dreh- oder Relativposition zwischen Trokarhülse und Trokardorn in der Schiebeendstellung gesichert, in der die exzentrische Trokardornspitze im Bereich des distalen, dem Patienten zugewandten Endes der Trokarhülse angeordnet ist. Dabei kann es vorteilhaft sein, wenn diese Drehsicherungsmittel arretierbar sind, so daß die beiden Instrumententeile vom Operateur nicht in ihrer Schiebeendstellung gehalten werden müssen.

Dabei ist es zweckmäßig, wenn am proximalen Endbereich des Trokardorns oder der Trokarhülse wenigstens ein Drehsicherungsvorsprung vorgesehen ist, der zumindest in einer Schiebeendstellung in eine Drehsicherungs-Ausnehmung des jeweils anderen Instrumenten-Teiles eingreift. Beispielsweise kann dieser Drehsicherungsvorsprung an dem als Handhabe ausgebildeten proximalen Endbereich des Trokardorns vorgesehen sein, wobei dieser Drehsicherungsvorsprung in der Schiebeendstellung in ,einer am benachbarten proximalen Ende der Trokarhülse vorgesehene Drehsicherungs-Ausnehmung eingreift.

Der Trokardorn des erfindungsgemäßen Operationsinstrumentes weist vorzugsweise eine an einen Oberflächenbereich einer Kegelform im wesentlichen formangepaßte Trokardornspitze auf. Dabei kann der Trokardorn auch eine zu seiner Trokardornspitze konkav oder konvex zulaufende Oberfläche aufweisen.

Eine andere weiterbildende Ausführungsform gemäß der Erfindung, betrifft ein Operationsinstrument der eingangs erwähnten Art, bei dem zwischen dem Trokardorn und der Trokarhülse zumindest ein Gaskanal vorgesehen ist, der im Bereich einer Gaszutritts- oder Gasauslaßöffnung der Trokarhülse mündet. Mit Hilfe eines solchen Gaskanals zwischen dem Trokardorn und der Trokarhülse kann bei den herkömmlichen Einstichverfahren durch einen entsprechenden Gasaustritt an der Gasauslaßöffnung der Trokarhülse festgestellt werden, wenn das Operationsinstrument das mittels der Insuflationsnadel aufgeweitete Peritoneum des Patienten durchstochen trat. Bei einem solchen Operationsinstrument ist erfindungsgemäß vorgesehen, daß der Gasauslaßöffnung der Trokarhülse in Ausströmrichtung eine Signalpfeife nachgeschaltet ist. Mit Hilfe einer solchen Signalpfeife kann wesentlich sicherer festgestellt werden, ob das Operatinsinstrument das Bauchfell durchstoßen hat und ob das zuvor in das Bauchinnere eingeführte Gas über die Gasauslaßöffnung der Trokarhülse entweicht. Da bei dem Ertönen eines Signaltons der Signalpfeife ein weiterer Vorschub des Operationsinstrumentes in das Bauchinnere abgebrochen oder zumindest abgebremst werden kann, werden ebenfalls unbeabsichtigte Verletzungen des Patienten vermieden.

Dabei ist es vorteilhaft, wenn der Trokardorn zwischen seinen beiden Endbereichen einen Teilbereich reduzierten Querschnitts aufweist, der zwischen sich und der Trokarhülsen-Innenwand einen Gaskanal begrenzt und wenn am distalen Endbereich des Trokardorns ein vorzugsweise nutförmiger Kanalabschnitt vorgesehen ist, der von der Trokardornspitze zum reduzierten Querschnittsbereich des Trokardorns führt. Dieser, bei einem vergleichsweise großen Teilbereich seine Längserstreckung mit einem reduzierten Querschnitt ausgebildete Trokardorn, läßt sich besonders gut reinigen.

Dabei kann der im Querschnitt reduzierte Teilbereich des Trokardorns beispielsweise einen kreuzförmigen Querschnitt aufweisen oder bloß aus drei oder vier Verbindungsstegen gebildet sein, welche das eventuell massiv ausgestaltete Trokardornvorderteil mit dem als Handhabe ausgebildeten proximalen Endbereich des Trokardorns verbindet.

Weitere Merkmale der Erfindung ergeben sich aus der folgenden Beschreibung erfindungsgemäßer Ausführungsbeispiele in Verbindung mit den Ansprüchen sowie der Zeichnung. Die einzelnen Merkmale können je für sich oder zu mehreren bei einer Ausführungsform gemäß der Erfindung verwirklicht sein.

Es zeigt:
- Fig.1: einen Trokardorn in einer perspektivischen Darstellung, der eine zu seiner Trokardorn-Längsachse exzentrisch angeordnete Trokardornspitze aufweist,
- Fig.2: eine dem Trokardorn aus Fig.1 zugeordnete Trokarhülse in einer perspektivischen Darstellung, wobei diese Trokarhülse sowie der Trokardorn ein Operationsinstrument bilden,
- Fig.3: ein Operationsinstrument, ähnlich dem aus Fig.1 und 2, wobei der Trokardorn dieses Operationsinstrumentes in die Trokarhülse eingeschoben ist.

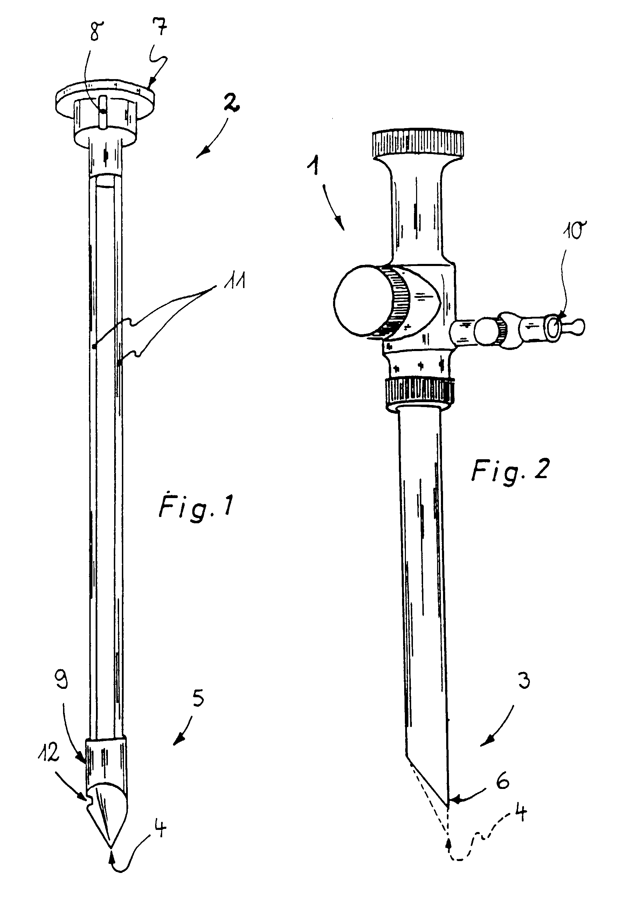

In Fig.2 ist eine Trokarhülse 1 dargestellt, in der ein in Fig.1 gezeigter Trokardorn 2 verschieblich geführt ist. Trokarhülse 1 und Trokardorn 2 bilden ein Operationsinstrument, das in der minimal-invasiven Chirurgie einsetzbar ist und dazu in das Körperinnere eines Patienten eingestochen werden kann.

Zum Einstechen des Operationsinstrumentes steht der in die Trokarhülse 1 eingeführte Trokardorn 2 in einer Schiebeendstellung über den distalen Endbereich 3 der Trokarhülse 1 über. Dabei ist die am distalen Endbereich 3 der Trokarhülse 1 vorgesehene Hülsenöffnung gegenüber der Trokarhülsen-Längsachse abgeschrägt, um das Nachschieben der Trokarhülse 1 beim Einstechen des Operationsinstrumentes zu erleichtern.

Die Trokardornspitze 4 des Trokardorns 2 ist exzentrisch zur Trokardorn-Längsachse angeordnet. Wie aus Fig.1 deutlich wird, ist der distale Endbereich 5 des Trokardorns 2 im wesentlichen als eine Verschneidung oder Verschneidungskurve zwischen einem etwa in Hülsen-Längsrichtung orientierten Zylinder und einer Kegelform ausgebildet. Die Kegelspitze dieser Kegelform, die hier gleichzeitig auch die Trokardornspitze 4 bildet, ist etwa auf dem Trokardorn-Umfang angeordnet und läuft punktförmig spitz zu.

Durch die exzentrische Ausgestaltung der Trokardornspitze 4 und deren Anordnung im Bereich des dem Patienten zugewandten vordersten Hülsenendes 6 der Trokarhülse 1 steht die in Fig.2 gestrichelt angedeutete Trokardornspitze 4 dennoch nur geringfügig über die distale Hülsenöffnung der Trokarhülse 1 über. Damit wird der Abstand zwischen dem vordersten Hülsenende 6 der Trokarhülse 1 und der benachbarten Trokardornspitze 4 wesentlich verringert und das Operationsinstrument muß weniger weit durch die Bauchdecke vorgeschoben werden, bis die Trokarhülse das an sich zähe und zeltförmig zurückweichende Bauchfell durchstoßen hat.

Am proximalen und als Handhabe 7 ausgebildeten Endbereich des Trokardorns 2 ist ein Drehsicherungsvorsprung 8 vorgesehen, der in der Schiebeendstellung in eine korrespondierende - hier aber nicht dargestellte - Drehsicherungs-Ausnehmung eingreift, die am benachbarten proximalen Ende der Trokarhülse 1 vorgesehen ist.

Wie aus Fig.1 deutlich wird, ist das distale Trokardornvorderteil 9 als massives Bauteil ausgeführt und weist einen im wesentlichen zylindrischen Außenumfang auf, der dem lichten Durchmesser der Trokarhülse 1 entspricht. In dem zwischen der Handhabe 7 des Trokardorns 2 und seiner Trokardornspitze 4 bzw. dem Trokardornvorderteil 9 angeordneten Teilbereich hat der Trokardorn 2. einen reduzierten Querschnitt, der zwischen sich und der Trokarhülsen-Innenwand einen Gaskanal begrenzt, welcher in der Schiebeendstellung in einer Gaszufuhr- und Gasaustrittsöffnung 10 der Trokarhülse 1 mündet.

In diesem reduzierten Querschnittsbereich des Trokardorns 2 sind lediglich vier Verbindungsstäbe 11 vorgesehen, die dessen Handhabe 7 mit dem Trokardornvorderteil 9 verbinden. Von diesen vier Verbindungsstäben 11 sind in Fig.1 nur zwei Stäbe 11 zu sehen.

Am distalen Endbereich 5 des Trokardorns 2 ist ein nutförmiger Kanalabschnitt 12 vorgesehen, der von der Trokardornspitze 4 zum reduzierten Querschnittsbereich des Trokardorns 2 führt.

Hat nach dem Einstechen des Operationsinstrumentes in der bekannten Weise das Trokardornvorderteil 9 das Bauchfell des durch Gasinsuflation erweiterten Bauchinneren durchstoßen, wird dies dem Operateur angezeigt, wenn nämlich das Gas von dort über den Kanalabschnitt 12, den von den Verbindungsstäben 11 freigehaltenen Gaskanal sowie die Gasaustrittsöffnung 10 der Trokarhülse 1 zischend nach außen entweicht.

Dabei kann es zweckmäßig sein, wenn der Gasauslaßöffnung 10 der Trokarhülse 1 in Ausströmrichtung eine Signalpfeile nachgeschaltet ist. Beim Ertönen eines Signaltones der Signalpfeife kann der Operateur den Einstichvorgang abbremsen oder abbrechen, noch bevor er versehentlich innere Organe oder Gefäße des Patienten verletzt.

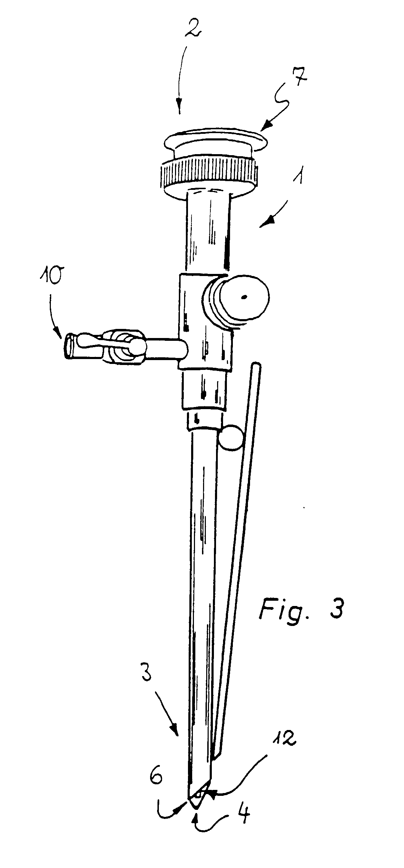

In Fig.3 ist ein Operationsinstrument gezeigt, dessen Trokardorn-spitze 4 ebenfalls exzentrisch zur Trokardorn-Längsachse angeordnet und an den Oberflächenbereich einer Kegelform angepaßt ist. Dabei ist die Trokardornspitze 4 mit Abstand von der Trokardorn-Längsachse einerseits und vom Trokardorn-Umfang andererseits innerhalb des Trokardornquerschnitts vorgesehen. Diese in Fig.2 dargestellte Anordnung der Trokardornspitze 4 erlaubt es, insbesondere bei kleineren Trokarhülsen-Durchmessern eine flachere Spitzenebene vorzusehen.

Wie Fig.3 zeigt, ist die Trokardornspitze 4 hier geringfügig abgerundet, um die Verletzungsgefahr bei dem in Fig.3 dargestellten Operationsinstrument noch zusätzlich zu reduzieren.

Zusammenfassend läßt sich feststellen, daß durch die exzentrische Ausgestaltung der Trokardornspitze 4 gegenüber der Trokardorn-Längsachse und durch die Anordnung dieser Trokardornspitze 4 am distalen, dem Patienten zugewandten Ende der Trokarhülse 1 das mit dem Einstechen des Operationsinstrumentes verbundene Verletzungsrisiko für den Patienten wesentlich reduziert wird.

## Patentansprüche

1. Operationsinstrument, das mit seinem distalen Endbereich in das Körperinnere eines Patienten einzuführen oder einzustechen ist und eine äußere Trokarhülse sowie einen darin einführbaren und verschieblich geführten Trokardorn aufweist, wobei der Trokardorn in einer Schiebeendstellung mit seiner distalen Trokardornspitze über den zur Hülsen-Längsachse schrägen Endbereich der Trokarhülse übersteht, dadurch gekennzeichnet, daß die Trokardorn-spitze (4) gegenüber der Trokardorn-Längsachse exzentrisch ausgebildet und im Bereich des vordersten dem Patienten zugewandten Endes (6) der Trokarhülse (1) angeordnet ist.

2. Operationsinstrument nach Anspruch 1, dadurch gekennzeichnet, daß der distale Endbereich (5) des Trokardorns im wesentlichen als eine Verschneidung oder Verschneidungskurve zwischen einem etwa in Hülsen-Längsrichtung orientierten Zylinder und einer Kegelform ausgebildet ist.

3. Operationsinstrument nach Anspruch 1 oder 2, dadurch gekennzeichnet, daß die Trokardornspitze (4) innerhalb des Trokardornquerschnitts und vorzugsweise mit Abstand von der Trokardorn-Längsachse einerseits und dem Trokardorn-Umfang andererseits angeordnet ist.

4. Operationsinstrument nach einem der Ansprüche 1 bis 3, dadurch gekennzeichnet, daß die Kegelspitze außerhalb des Trokardornquerschnitts oder etwa auf dem Trokardorn-Umfang angeordnet ist.

5. Operationsinstrument nach einem der Ansprüche 1 bis 4, dadurch gekennzeichnet, daß die Trokardornspitze schneidenförmig ausgebildet oder endseitig geringfügig abgerundet ist.

6. Operationsinstrument nach einem der Ansprüche 1 bis 5, dadurch gekennzeichnet, daß am Trokardorn (2) und/oder an der Trokarhülse (1) wenigstens ein in zumindest einer Schiebeendstellung wirksames Drehsicherungsmittel zur Sicherung der Relativposition von Trokarhülse (1) und Trokardorn (2) in deren Umfangsrichtung vorgesehen ist.

7. Operationsinstrument nach einem der Ansprüche 1 bis 6, dadurch gekennzeichnet, daß am proximalen Endbereich des Trokardorns (2) oder der Trokarhülse (1) wenigstens ein Drehsicherungsvorsprung (8) vorgesehen ist, der zumindest in einer Schiebeendstellung in eine Drehsicherungs-Ausnehmung des jeweils anderen Instrumenten-Teiles (1,2) eingreift.

8. Operationsinstrument nach einem der Ansprüche 1 bis 7, dadurch gekennzeichnet, daß der Trokardorn (2) eine zu seiner Trokardornspitze (4) konkav oder konvex zulaufende Oberfläche hat.

9. Operationsinstrument nach einem der Ansprüche 1 bis 8, wobei zwischen dem Trokardorn und der Trokarhülse zumindest ein Gaskanal vorgesehen ist, der im Bereich einer Gaszutritts- oder Gasauslaßöffnung der Trokarhülse mündet, dadurch gekennzeichnet, daß der Gaszutritts- oder Gasauslaßöffnung (10) der Trokarhülse (1) in Ausströmrichtung eine Signalpfeife nachgeschaltet ist.

10. Operationsinstrument nach einem der Ansprüche 1 bis 9, dadurch gekennzeichnet, daß der Trokardorn (2) zwischen seinen beiden Endbereichen einen Teilbereich reduzierten Querschnitts aufweist, der zwischen sich und der Trokarhülsen-Innenwand einen Gaskanal begrenzt und daß am distalen Endbereich (5) des Trokardorns (2) ein vorzugsweise nutförmiger Kanalabschnitt (12) vorgesehen ist, der von der Trokardornspitze (4) zum reduzierten Querschnittsbereich des Trokardorns (2) führt.

## Claims

1. Surgical instrument the distal end region of which is to be introduced or pierced into the inside of a patient's body and which has an outer trocar sleeve and a trocar mandrel which can be inserted in the sleeve and is movably guided therein, wherein the distal tip of the trocar mandrel in an end position projects beyond the end region of the trocar sleeve which is inclined relative to the longitudinal axis of the sleeve, characterised in that the tip of the trocar mandrel (4) is eccentrically formed relative to the longitudinal axis of said mandrel and is disposed in the region of the front end (6) of the trocar sleeve (1) nearest the patient.

2. Surgical instrument according to claim 1, characterised in that the distal end region (5) of the trocar mandrel is constructed essentially as a transition or transitional curve between a cylinder oriented substantially in the longitudinal direction of the sleeve and a conical shape.

3. Surgical instrument according to claim 1 or 2, characterised in that the tip (4) of the trocar mandrel is disposed inside the cross section of the mandrel and preferably at a spacing from the longitudinal axis of the mandrel, on the one hand, and the circumference of the mandrel, on the other hand.

4. Surgical instrument according to one of claims 1 to 3, characterised in that the tip of the cone is disposed outside the cross section of the trocar mandrel or substantially on the circumference of the trocar mandrel.

5. Surgical instrument according to one of claims 1 to 4, characterised in that the tip of the trocar mandrel is shaped like a blade or slightly rounded off at the end.

6. Surgical instrument according to one of claims 1 to 5, characterised in that on the trocar mandrel (2) and/or on the trocar sleeve (1) is provided at least one anti-rotation device which is effective in at least one end position, for securing the relative positions of the trocar sleeve (1) and mandrel (2) in the circumferential direction thereof.

7. Surgical instrument according to one of claims 1 to 6, characterised in that at the proximal end region of the trocar mandrel (2) or trocar sleeve (1) is provided at least one anti-rotation projection (8) which engages in an anti-rotation recess in the other part of the instrument (1, 2) at least in one end position.

8. Surgical instrument according to one of claims 1 to 7, characterised in that the trocar mandrel (2) has a surface which converges in concave or convex manner towards the mandrel tip (4).

9. Surgical instrument according to one of claims 1 to 8, wherein at least one gas channel is provided between the trocar mandrel and trocar sleeve, this channel ending in the region of a gas inlet or outlet opening of the trocar sleeve, characterised in that a signal whistle is located downstream of the gas inlet or outlet opening (10) of the trocar sleeve (1) in the direction of outflow.

10. Surgical instrument according to one of claims 1 to 9, characterised in that the trocar mandrel (2) has a portion of reduced cross section between its two end regions, which delimits a gas channel between itself and the inner wall of the trocar sleeve, and in that at the distal end region (5) of the trocar mandrel (2) there is a preferably groove-shaped channel portion (12) which leads from the tip (4) of the trocar mandrel to the area of reduced cross section of the trocar mandrel (2).

## Revendications

1. Instrument chirurgical, qui peut être introduit ou enfoncé par sa région terminale distale à l'intérieur du corps d'un patient et qui présente une canule extérieure de trocart et un poinçon de trocart pouvant être introduit dans cette canule et guidé en coulissement dans cette canule, le poinçon de trocart, dans une position finale de coulissement, dépassant par sa pointe distale de poinçon de trocart de la région terminale de la canule de trocart qui est en oblique par rapport à l'axe longitudinal de la canule, **caractérisé** en ce que la pointe (4) du poinçon de trocart est réalisée excentrique par rapport à l'axe longitudinal du poinçon de trocart, et elle est disposée dans la région de l'extrémité (6) la plus avant, tournée vers le patient, de la canule de trocart (1).

2. Instrument chirurgical selon la revendication 1, **caractérisé** en ce que la région terminale distale (5) du poinçon de trocart est réalisée essentiellement sous la forme d'une intersection ou courbe d'intersection entre un cylindre orienté environ dans la direction longitudinale de la canule et une forme de cône.

3. Instrument chirurgical selon la revendication 1 ou 2, **caractérisé** en ce que la pointe (4) du poinçon de trocart est disposée à l'intérieur de la section du poinçon de trocart, et de préférence à distance de l'axe longitudinal du poinçon de trocart d'une part et de la circonférence du poinçon de trocart d'autre part.

4. Instrument chirurgical selon une des revendications 1 à 3, **caractérisé** en ce que la pointe du cône est disposée en dehors de la section du poinçon de trocart ou environ sur la circonférence du poinçon de trocart.

5. Instrument chirurgical selon une des revendications 1 à 4, **caractérisé** en ce que la pointe du poinçon de trocart est réalisée en forme de tranchant ou est légèrement arrondie à l'extrémité.

6. Instrument chirurgical selon une des revendications 1 à 5, **caractérisé** en ce qu'au moins un moyen de blocage en rotation, actif dans au moins une position finale de coulissement, est prévu sur le poinçon de trocart (2) et/ou sur la canule de trocart (1) pour bloquer la position relative de la canule de trocart (1) et du poinçon de trocart (2) dans leur direction circonférentielle.

7. Instrument chirurgical selon une des revendications 1 à 6, **caractérisé** en ce qu'au moins une saillie (8) de blocage en rotation est prévue sur la région terminale proximale du poinçon de trocart (2) ou de la canule de trocart (1), saillie qui s'engage, au moins dans une position finale de coulissement, dans un évidement de blocage en rotation de l'autre partie respective (1, 2) de l'instrument.

8. Instrument chirurgical selon une des revendications 1 à 7, **caractérisé** en ce que le poinçon de trocart (2) possède une surface d'allure concave ou convexe en direction de sa pointe (4) de poinçon de trocart.

9. Instrument chirurgical selon une des revendications 1 à 8, au moins un canal de gaz étant prévu entre le poinçon de trocart et la canule de trocart, canal qui débouche dans la région d'une ouverture d'entrée de gaz ou de sortie de gaz de la canule de trocart, **caractérisé** en ce que l'ouverture (10) d'entrée de gaz ou de sortie de gaz de la canule de trocart (1) est suivie, dans la direction de flux sortant, d'un sifflet de signalisation.

10. Instrument chirurgical selon une des revendications 1 à 9, **caractérisé** en ce que le poinçon de trocart (2) présente, entre ses deux régions terminales, une région partielle de section réduite qui délimite entre elle et la paroi intérieure de la canule de trocart un canal de gaz, et en ce qu'un tronçon de canal (12), de préférence en forme de rainure, est prévu dans la région terminale distale (5) du poinçon de trocart (2), tronçon qui mène de la pointe (4) du poinçon de trocart à la région de section réduite du poinçon de trocart (2).
